(19) Europäisches Patentamt / European Patent Office / Office européen des brevets

(11) **EP 0 986 546 B1**

(12) **EUROPEAN PATENT SPECIFICATION**

(45) Date of publication and mention
of the grant of the patent:
**28.03.2007 Bulletin 2007/13**

(51) Int Cl.:
***C07D 251/60*** (2006.01)

(86) International application number:
**PCT/NL1998/000280**

(21) Application number: **98923216.0**

(22) Date of filing: **15.05.1998**

(87) International publication number:
**WO 1998/055465 (10.12.1998 Gazette 1998/49)**

(54) **CRYSTALLINE MELAMINE**

KRISTALLINES MELAMIN

MELAMINE CRISTALLINE

(84) Designated Contracting States:
**AT DE ES FR GB IT NL SE**

(30) Priority: **02.06.1997 NL 1006191**
**25.06.1997 EP 97201940**

(43) Date of publication of application:
**22.03.2000 Bulletin 2000/12**

(60) Divisional application:
**05076469.5 / 1 604 985**

(73) Proprietor: **DSM IP Assets B.V.**
**6411 TE Heerlen (NL)**

(72) Inventor: **TJIOE, Tjay, Tjien**
**NL-6136 BR Sittard (NL)**

(56) References cited:
**EP-A- 0 808 836       WO-A-96/20182**
**WO-A-96/20183       WO-A-97/20826**
**US-A- 4 565 867       US-A- 5 514 796**

<u>Remarks:</u>
The file contains technical information submitted after the application was filed and not included in this specification

**Description**

The invention relates to crystalline melamine, more in particular to multicrystalline melamine powder.

Various processes have been developed for the preparation of melamine on an industrial scale. Some methods ultimately involve the crystallization of melamine from an aqueous solution or the condensation of melamine from its gaseous phase. Another method involves synthesizing melamine at high pressure (7-25 MPa), producing a melamine melt, and spraying the melamine melt into an ammonia atmosphere where it is solidified and cooled, thereby forming a crystalline powder of sufficient purity without additional purification steps.

Crystalline melamine obtained by crystallization from an aqueous solution is very pure, but the melamine crystals are relatively large, so that the rate of dissolution and the reactivity in a solvent such as, for example, water or a water/formaldehyde mixture are not optimal. For these reasons, the melamine obtained in this way is usually ground to afford more suitable particle sizes. Smaller particles, however, including those produced by grinding, have a higher reactivity but a lower bulk density and poor flow characteristics. As a result, an optimal product in terms of combination of reactivity, bulk density and flow characteristics is not obtained. Crystalline melamine obtained by condensation from the gaseous phase is very fine and consequently also has relatively poor flow characteristics.

Crystalline melamine obtained by spraying a melamine melt in an ammonia atmosphere is a multicrystalline and reactivity characteristics as well as reasonable flow characteristics. In practice, however, this melamine powder has been found to contain high impurity concentrations (in particular melam). Moreover, the color is unsatisfactory for a number of applications, particularly melamine-formaldehyde resins used in laminates and/or coatings. A method has been proposed of spraying the melamine at a relatively high pressure to reduce the melam concentration and has been described in EP-A-747366.

EP-A-747366 describes a high-pressure process for preparing melamine from urea in which urea is pyrolyzed in a reactor, operating at a pressure of from 10.34 to 24.13 MPa and a temperature of from 354 to 454°C, to produce a reactor product. This reactor product, containing liquid melamine, $CO_2$, and $NH_3$ and is transferred under pressure as a mixed stream to a separator.

In this separator, which is kept at virtually the same pressure and temperature as the reactor, the reactor product is separated into a gaseous stream and a liquid stream. The gaseous stream contains primarily $CO_2$ and $NH_3$ waste gases and melamine vapor. The liquid stream mainly comprises a melamine melt. The gaseous stream is transferred to a scrubber unit, while the liquid stream is transferred to a product-cooling unit.

In the scrubber unit, operated at temperature and pressure conditions nearly identical to the reactor conditions, the gaseous stream is scrubbed with molten urea. The heat transfer achieved in the scrubber unit both preheats the molten urea and cools the gaseous stream to a temperature from 177 to 232°C. The molten urea also scrubs the gaseous stream to remove the melamine vapor from the waste gases. The preheated molten urea, along with the melamine that was scrubbed from the $CO_2$ and $NH_3$ waste gases, is then fed into the reactor.

In the product-cooling unit, the melamine melt is cooled and solidified with a liquid cooling medium to produce a solid high purity melamine product without the need for additional purification. The preferred liquid cooling medium is one that forms a gas at the temperature of the melamine melt and at the pressure in the product-cooling unit. EP-A-747366 identifies liquid ammonia as the preferred liquid cooling medium with the pressure in the product-cooling unit being above 41.4 bar. Although according to EP-A-747366 the purity of the solid melamine product obtained using the disclosed process was greater than 99 wt%, this degree of purity has proven difficult to maintain continuously on a commercial scale. It has also been found that products made in this way may be slightly yellow in color. This is a drawback, in particular with melamine-formaldehyde resins which are used in laminates and/or coatings.

Examples of other publications directed at the lowering of the melam concentration include WO-A-96/20182, WO-A-96/20183, US-A-4565867 and WO-A-96/23778. These publications do not, however, address other characteristics of the melamine.

The object of the present invention is to obtain improved crystalline melamine powder, where melamine is to be obtainable with a high degree of purity as a dry powder directly from the melamine melt coming from a high pressure reactor. More particularly, the object of the present invention is to obtain crystalline melamine powder with a high dissolution rate in water, acceptable flow characteristics, high purity, and good color.

The invention relates to multicrystalline melamine powder having the following properties:

$d_{90}$: 50-150 $\mu$m; $d_{50}$ < 50 $\mu$m
bulk density (loose): 430-570 kg/m$^3$
color APHA less than 17
with a purity of > 98.5 wt% of melamine
with less than 1 wt% of melam.

This product differs from melamine powder obtainable from gaseous melamine in terms of the larger particles, as a result of which the melamine powder according to the invention has better flow characteristics and higher bulk density. The product according to the invention differs from melamine crystallized from water in terms of a higher reactivity (given an identical particle size distribution) and a different combination of particle size distribution, flow characteristics and bulk density.

The particle size distribution was measured with a laser diffraction technique applied to the dry powder in air (Sympatec); the bulk density (loosely dumped) was measured in accordance with ASTM 1895.

Preferably, the $d_{90}$ is between 70 and 120 $\mu$m and the $d_{50}$ is < 40 $\mu$m, with the bulk density being between 450 and 550 kg/m$^3$ and more preferably between 470 and 530 kg/m$^3$.

One customary method for determining the color of melamine is by so-called APHA colorimetry. This involves the preparation of a formaldehyde-melamine resin with an F/M ratio of about 3.0, using a formaldehyde solution with 35 wt.% formaldehyde, 7.5-11.2 wt% methanol, and 0.028 wt% of an acid (particularly formic acid). The theoretical solids content of the solution thus prepared is about 56 wt.%. A 25 gram sample of melamine is then slowly dissolved in 51 grams of the above formaldehyde solution while the mixture is being heated till 85°C. After approximately 3 minutes the melamine is dissolved. To this solution is added 2 ml of a 2.0 mol/l sodiumcarbonate solution after which the solution is stirred for 1-2 minutes and rapidly cooled down to 40°C. The color of the resulting mixture is than analyzed with a Hitachi U100 spectrofotometer using a 4 cm glass cell. Extinction measurements at wavelengths of 380 nm and 640 nm are carried out with water as a reference. The APHA color is calculated with the following formula:

$$APHA = f * (E\ 380 - E\ 640)$$

with

E 380 = extinction at 380 nm
E 640 = extinction at 640 nm
f = calibrationfactor.

The calibrationfactor [f] is determined by extinction measurements at 380 nm with standard solutions of cobaltchloride and potassium hexachloroplatinate. A 500 APHA standard solutions contains 1.245 gram potassium hexachloroplatinate (IV), 1.000 gram cobalt (II) chloride and 100 ml 12 M hydrochloricacid solution in 1 liter standard solutions. From this standard solutions, calibration solutions are prepared with 10 and 20 APHA. The calibrationfactor f is calculated with the following formula:

$$f = APHA\ (standard\ solution)\ /\ E\ 380$$

The color of the melamine obtained when using melamine prepared according to the present invention is lower than 17 APHA, preferably lower than 15 APHA and most preferably lower than 12 APHA.

The yellowness of the product can be measured in accordance with the Hunterlab-C.I.E. method. According to this method, 60 g of melamine powder is introduced into a cuvette of a Hunterlab ColorQUEST® spectrophotometer. The measurement is carried out in accordance with the Hunterlab method C.I.E., values being determined for L', a' and b'. In the Hunterlab C.I.E. method, the value of b' is a measure of the blue-yellow shift, the b' value being positive if the product is yellow and negative if the product is blue. The greater the positive value, the more yellow the product.

The color of the melamine powder preferably has a b' value of less than 1, more preferably less than about 0.8, because resins produced from this melamine are entirely water-white.

The concentration of melam in this melamine powder is preferably less than 0.5 wt%, and more preferably less than 0.3 wt%. The purity of the melamine is preferably greater than 99 wt%, more preferably between 99.5 and 99.8 wt%, purity that approaches the purity of melamine crystallized from water.

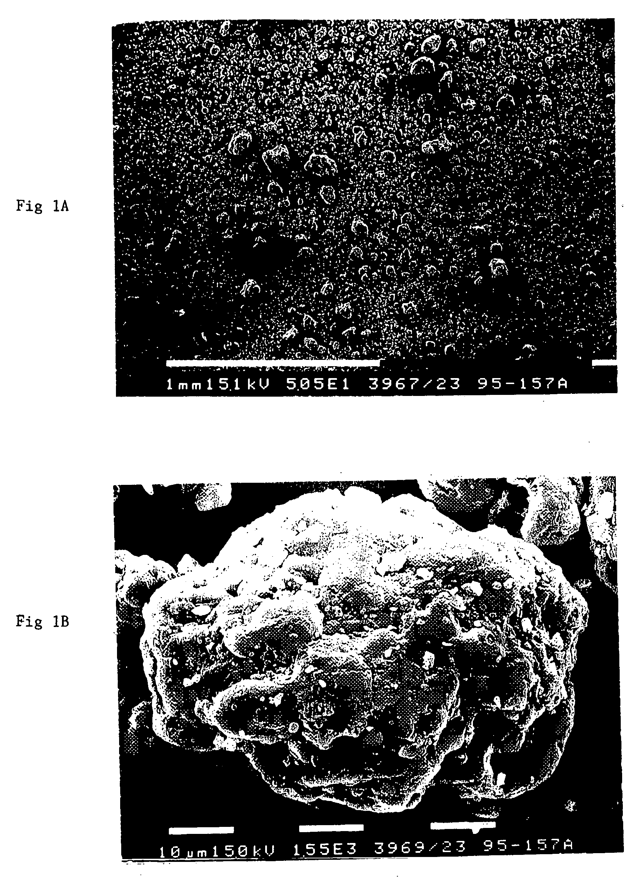

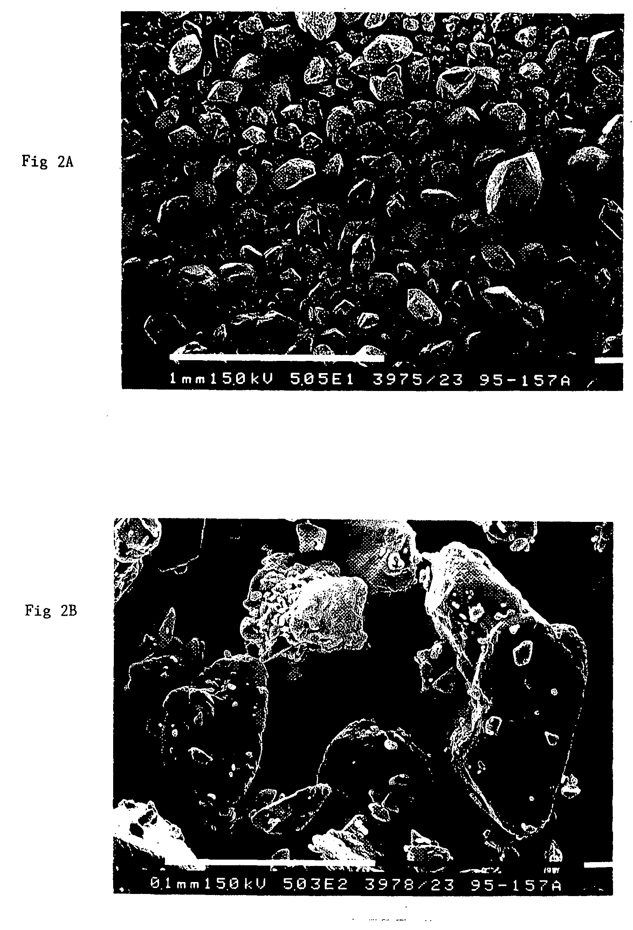

The melamine powder according to the invention consists of multicrystalline particles. This means that the larger particles (> 20 $\mu$m) are composed of a multiplicity of crystals. On a scanning electron micrograph these particles can be clearly distinguished from melamine crystallized from water. The particles according to the invention have a cauliflower-like structure. The melamine crystallized from water, in contrast, contains a substantial amount of crystals having a crystal size greater than 50 $\mu$m. On the SEM pictures the crystallographic crystal faces (large relatively flat areas) are clearly discernible in of melamine crystallized from water. The differences in crystal structure resulting from the different methods can also be seen in Figures 1 and 2. Figure 1 comprises SEM pictures (Figure 1A: 50x; and Figure 1B: 1500x) of particles made according to the invention and exhibiting a so-called cauliflower structure. Figure 2, however, comprises SEM pictures of melamine crystallized from water (Figure 2A: 50x and Figure 2B: 500x). The photographs of both products were produced using a Philips SEM 515 at an accelerating voltage of 15 kV.

The applicant has now also found that melamine having continuously high purity can be produced directly from the melamine melt from a high pressure melamine reactor. The melamine melt, having a temperature between the melting point of melamine and 450°C, is sprayed via a spraying means into a cooling vessel. In the cooling vessel the melamine melt is cooled by means of an evaporating cooling medium in an ammonia environment at an ammonia pressure of 4.5-25 MPa, the melamine melt being converted into melamine powder having a temperature of between 200°C and the solidification point of melamine. The melamine powder is then cooled further to a temperature below 50°C, the powder mechanically agitating over at least part of the cooling range and being cooled directly or indirectly, the ammonia pressure being released at a temperature below 270°C.

Melamine powder has poor flow and fluidization characteristics and a low temperature equalization coefficient (poor thermal conductivity). Standard cooling methods such as a fluidized bed or a packed moving bed can therefore not readily be implemented on a commercial scale. The applicant has found, however, that the color of the melamine powder, in particular, is adversely affected if the melamine remains at a high temperature for too long. Effective control of the residence time at high temperature has therefore proved critical. It is therefore important to be able to cool the melamine powder effectively.

Surprisingly, it proved possible to cool melamine powder, despite its poor flow and thermal conductivity characteristics, by utilizing mechanical agitation coupled with direct and indirect cooling. The term indirect cooling describes those instances in which the mechanically agitated melamine powder contacts a cooled surface. The term direct cooling describes those instances in which the mechanically agitated melamine powder contacts a cooling medium such as ammonia or an airstream. A combination of both direct and indirect cooling mechanisms is obviously also possible.

The melamine powder formed by spraying the melamine melt into the solidification vessel is held under an ammonia pressure of 4.5-25 MPa at a temperature above 200°C for a contact time. The duration of this contact time is preferably between 1 minute and 5 hours, more preferably between 5 minutes and 2 hours. During this contact time, the temperature of the melamine product can remain virtually constant or it may be cooled to a temperature above 200°C, preferably above 240°C, or, most preferably, above 270°C. The melamine product may be cooled in the solidification vessel or in a separate cooling vessel.

Preferably, the residence time at a temperature above 200°C is such that the discoloration is less than the discoloration corresponding to a b' of about 1. At lower temperatures a longer residence time is permitted before yellowing exceeds the specification. At higher temperatures a shorter residence time is permitted. The advantage of the method according to the present invention is that a powdered melamine is obtained with a purity which is continuously above 98.5 wt%, and preferably above 99 wt%. The high purity melamine produced according to the present invention is suitable for virtually any melamine application, including melamine-formaldehyde resins used in laminates and/or coatings.

The preparation of melamine preferably uses urea as the raw material, the urea being fed into the reactor as a melt and reacted at elevated temperature and pressure. Urea reacts to form melamine, and the by-products $NH_3$ and $CO_2$, according to the following reaction equation:

$$6 \, CO(NH_2)_2 \rightarrow C_3N_6H_6 + 6 \, NH_3 + 3 \, CO_2$$

The production of melamine from urea can be carried out at high pressure, preferably between 5 and 25 MPa, without the presence of a catalyst, at reaction temperatures between 325 and 450°C, and preferably between 350 and 425°C. The by-products $NH_3$ and $CO_2$ are usually recycled to an adjoining urea factory.

The above-mentioned objective of the invention is achieved by employing an apparatus suitable for the preparation of melamine from urea. An apparatus suitable for the present invention may comprise a scrubber unit, a reactor having either an integrated gas/liquid separator or a separate gas/liquid separator, possibly a post-reactor, a first cooling vessel, and possibly additional cooling vessels. When a separate gas/liquid separator is used, the pressure and temperature of the separator are virtually identical to the temperature and pressure in the reactor.

In an embodiment of the invention, melamine is prepared from urea in an apparatus comprising a scrubber unit, a melamine reactor having either an integrated gas/liquid separator or a separate gas/liquid separator, a first cooling vessel, and a second cooling vessel. In this embodiment, the urea melt is fed into a scrubber unit operating at a pressure of from 5 to 25 MPa, preferably from 8 to 20 MPa, and at a temperature above the melting point of urea. This scrubber unit may be provided with a cooling jacket or internal cooling bodies to provide additional temperature control.

As it passes through the scrubber unit, the urea melt contacts the reaction waste gases coming from the melamine reactor or the separate gas/liquid separator. The reaction gases mainly consist of $CO_2$ and $NH_3$ and may include melamine vapor. The urea melt scrubs the melamine vapor from the $CO_2$ and $NH_3$ waste gases and carries this melamine along back to the reactor. In the scrubbing process, the waste gases are cooled from the temperature of the reactor, i.e. from 350 to 425°C, to from 170 to 240°C, the urea being heated to from 170 to 240°C. The $CO_2$ and $NH_3$ waste gases are removed from the top of the scrubber unit and may, for example, be recycled to an adjoining urea factory, where they can be used as raw materials for the urea production.

The preheated urea melt is drawn off from the scrubber unit, together with the melamine scrubbed from the waste gases, and transferred to the high pressure reactor operating at pressures between 5 and 25 MPa, and preferably between 8 and 20 MPa. This transfer may be achieved using a high-pressure pump or, where the scrubber is positioned above the reactor, gravity, or a combination of gravity and pumps.

In the reactor, the urea melt is heated to a temperature between 325 and 450°C, preferably between about 350 and 425°C, under a pressure between 5 and 25 MPa, preferably between 8 and 20 MPa, to convert the urea into melamine, $CO_2$, and $NH_3$. In addition to the urea melt, a certain amount of ammonia can be metered into the reactor as, for example, a liquid or hot vapor. The additional ammonia, although optional, may serve, for example, to prevent the formation of condensation products of melamine such as melam, melem, and melon, or to promote mixing in the reactor. The amount of additional ammonia supplied to the reactor may be up to 10 moles ammonia per mole of urea, preferably up to 5 moles ammonia per mole of urea, and, most preferably, up to 2 moles of ammonia per mole of urea.

The $CO_2$ and $NH_3$ produced in the reaction, as well as any additional ammonia supplied, collect in the separation section, for example in the top of the reactor or in a separate gas/liquid separator positioned downstream of the reactor, and are separated from the liquid melamine. If a separate, downstream gas/liquid separator is used, it may be advantageous for additional ammonia to be metered into this separator. The amount of ammonia in this case is 0.01-10 moles of ammonia per mole of melamine, and preferably 0.1-5 moles of ammonia per mole of melamine. Adding additional ammonia to the separator promotes the rapid separation of carbon dioxide from the reactor product, thus preventing the formation of oxygen-containing by-products. As described above, the gas mixture removed from the gas/liquid separator may be passed to the scrubber unit in order to remove melamine vapor and preheat the urea melt.

The melamine melt, having a temperature between the melting point of melamine and 450°C, is drawn off from the reactor or from the downstream gas/liquid separator and sprayed into a cooling vessel to obtain the solid melamine product. Prior to spraying, however, the melamine melt may be cooled from the reactor temperature to a temperature closer to, but still above, the melting point of melamine.

The melamine melt will be drawn off from the reactor at a temperature preferably above 390°C, and more preferably above 400°C, and will be cooled at least 5°C, and preferably at least 15°C, before spraying into the cooling vessel. Most preferably the melamine melt will be cooled to a temperature that is 5-20°C above the solidification point of melamine. The melamine melt may be cooled in the gas/liquid separator or in a separate apparatus downstream of the gas/liquid separator. Cooling can take place by injection of a cooling medium, for example ammonia gas having a temperature below the temperature of the melamine melt, or by passing the melamine melt through a heat exchanger.

Furthermore, ammonia can be introduced into the melamine melt in such a way that a gas/liquid mixture is sprayed in the spraying means. In this case, the ammonia is introduced at a pressure above that of the melamine melt and preferably at a pressure between 10 and 45 MPa, more preferably between 15 and 30 MPa.

The residence time of the liquid melamine between the reactor and the spraying means is preferably greater than 10 minutes and more preferably greater than 30 minutes. The residence time will usually be less 7 hours and preferably less than 5 hours.

The melamine melt, possibly together with ammonia gas, is transferred to a spraying means where it is sprayed into a first cooling vessel to solidify the melamine melt and form a dry melamine powder. The spraying means is an apparatus by which the melamine melt stream is converted into droplets, by causing the melt to flow at high speed into the first cooling vessel. The spraying means may be a nozzle or valve. The outflow velocity of the melamine melt from the spraying means is, as a rule, greater than 20 m/s, and is preferably greater than 50 m/s.

The cooling vessel contains an ammonia environment and operates at a pressure of 4.5-25 MPa, preferably 6-11 MPa. The melamine powder thus formed having a temperature between 200°C and the solidification point of melamine, preferably between 240°C and the solidification point and, most preferably between 270°C and the solidification point. The melamine droplets from the spraying means are cooled by an evaporating cooling medium, for example, liquid ammonia, to produce melamine powder. The melamine melt may contain some portion of liquid ammonia with the remaining portion of the liquid ammonia being sprayed into the first cooling vessel.

After spraying, the melamine powder is cooled to a temperature below 50°C, the powder being agitated mechanically over at least part of the cooling range and being cooled directly or indirectly, and the ammonia pressure not being released until the melamine powder reaches a temperature below 270°C.

The melamine powder formed by spraying the melamine melt into the cooling vessel is held under an ammonia pressure of 4.5-25 MPa, preferably 6-11 MPa, at a temperature above 200°C for a contact time. The duration of this contact time is preferably between 1 minute and 5 hours, more preferably between 5 minutes and 2 hours. During this contact time, the temperature of the melamine product can remain virtually constant or it may be cooled to a temperature above 200°C.

The cooling range within which the melamine powder is mechanically agitated and is cooled directly or indirectly is preferably at least 35°C, in particular at least 60°C, because this allows a product to be obtained which has a different color.

If the melamine is sprayed and cooled to a temperature above 270°C, it is preferable for the means, for agitating mechanically the melamine powder and cooling it, to be used at an ammonia pressure of 4-25 MPa. However, if the melamine melt is sprayed and cooling takes place at the same time to a temperature below 270°C, preferably below 250°C, and most preferably to a temperature below 200°C, these means can be used at a lower pressure (0.05-0.2 MPa), which is advantageous because of lower investment costs.

The present method may be utilized in both batchwise and continuous processes. In the case of batchwise processing, two or more cooling vessels may be used with the melamine melt being sprayed sequentially into the various cooling vessels. Once a first cooling vessel contains the desired quantity of melamine powder, the spraying means for the first cooling vessel can be closed and the spraying means for the second cooling vessel opened. While the subsequent cooling vessels are being filled, the melamine powder in the first vessel can be treated further. In a continuous process, the liquid melamine will generally be sprayed in a first cooling vessel with the accumulating melamine powder being transferred into a second cooling vessel where the cooling step takes place. A hybrid of the batchwise and continuous methods may also be employed.

The melamine powder must be cooled from a temperature between the melting point of melamine and about 200°C to a temperature below 50°C. The melamine melt is preferably cooled during spraying to a temperature of 10 to 160°C below the solidification point. The melamine powder thus obtained is preferably cooled by at least 35°C, more preferably by at least 60°C, by the powder being agitated mechanically and being cooled directly or indirectly.

Cooling is effected with the aid of an apparatus provided with means for agitating powder mechanically and for cooling powder directly or indirectly. Examples of means for agitating powder mechanically include a screw and rotating drum, a rotating tray, rotating discs, rotating segment discs, rotating pipes and the like.

The melamine powder can be cooled indirectly by contact with the cooled surface (s) of the fixed and/or moving parts of the cooling apparatus. The fixed and/or moving surface(s) of the apparatus may, in turn, be cooled with a cooling fluid such as water or oil. The effective heat transfer coefficient of a suitable cooling apparatus for indirectly cooling melamine powder is preferably between 10 and 300 $W/m^2K$, based on the cooling area of the apparatus. Preference is also given to the use of a cooling apparatus which comprises means having a cooling area of 50-5000 $m^2$.

The powder can be cooled directly by a gaseous or evaporating cooling medium being injected into the vessel, preferably ammonia gas or ammonia liquid.

Obviously, it is also possible for a combination of direct and indirect cooling to be used.

This cooling apparatus is highly suitable both for cooling melamine powder at a high pressure (4-25 MPa) and at a low pressure (0.05-0.2 MPa) to a temperature of about 50-70 °C. Preferably, ammonia gas is completely removed (to an amount below 1000 ppm, preferably below 300 ppm, and, most preferably, below 100 ppm) by blowing air through the melamine powder.

The invention will be explained in more detail with reference to the following example.

Example

Melamine melt having a temperature of 402°C is introduced, via a spraying device, into a high-pressure vessel and cooled with liquid ammonia which is likewise sprayed into the vessel. The temperature in the vessel is 296°C. The high-pressure vessel is designed as a rotating drum provided with a wall which can be cooled, and provided with a gas inlet. The ammonia pressure in the vessel varies between 8.6 and 12 MPa. After 1 minute the product is cooled to ambient temperature. The cooling step to 200°C took 7 minutes. When the melamine powder had a temperature of about 180°C, all the $NH_3$ was released and air was metered into the vessel. The end product has the following properties:

$d_{90}$ = 106 $\mu$m; $d_{50}$ = 38 $\mu$m
bulk density (loose): 490 $kg/m^3$
color (APHA) : 10
99.2 wt% of melamine
0.4 wt% of melam
< 0.2 wt% of melem
concentration of ammonia 150 ppm

Comparative example

Melamine melt of 400°C, held in a tube under an ammonia pressure of 13.6 MPa, is rapidly cooled to ambient temperature by the closed tube being brought into contact with a mixture of ice and water. The end product contains 1.4 wt% of melam and 0.4 wt% of melem.

**Claims**

1.  Multicrystalline melamine powder having the following properties:

    - $d_{90}$: 50-150 $\mu$m; $d_{50}$ < 50 $\mu$m
    - bulk density (loose) 430-570 kg/m$^3$
    - color APHA less than 17
    - melamine: > 98.5 wt%
    - melam: < 1 wt%

2.  Multicrystalline melamine powder according to Claim 1, **characterized in that** the $d_{90}$ is between 70 and 120 $\mu$m and the $d_{50}$ is less than 40 $\mu$m.

3.  Multicrystalline melamine powder according to either one of Claims 1-2, **characterized in that** the color is lower than 15 APHA.

4.  Multicrystalline melamine powder according to any one of Claims 1-3. **characterized in that** the concentration of melam is less than 0.5 wt%.

5.  Multicrystalline melamine powder according to any one of Claims 1-4, **characterized in that** the purity of melamine is greater than 99 wt%.

6.  Multicrystalline melamine powder according to Claim 5, **characterized in that** the purity of melamine is between 99.5 and 99.8 wt%.

7.  Multicrystalline melamine powder according to any one of Claims 1-6, **characterized in that** the bulk density (loose) is between 450 and 550 kg/m$^3$.

8.  Multicrystalline melamine powder according to any one of Claims 1-7, **characterized in that** the yellowing of the melamine powder (b') is less than 1.

9.  Multicrystalline melamine powder according to Claim 8, **characterized in that** the yellowing (b') is less than 0.8.

**Patentansprüche**

1.  Multikristallines Melaminpulver mit den folgenden Eigenschaften:

    - $d_{90}$: 50-150 $\mu$m; $d_{50}$ < 50 $\mu$m
    - Schüttdichte (lose) 430-570 kg/m$^3$
    - Farbe APHA weniger als 17
    - Melamin: > 98,5 Gew.-%
    - Melam: < 1 Gew.-%.

2.  Multikristallines Melaminpulver nach Anspruch 1, **dadurch gekennzeichnet, daß** der $d_{90}$-Wert zwischen 70 und 120 $\mu$m liegt und der $d_{50}$-Wert weniger als 40 $\mu$m beträgt.

3.  Multikristallines Melaminpulver nach Anspruch 1 oder 2, **dadurch gekennzeichnet, daß** die Farbe weniger als 15 APHA beträgt.

4.  Multikristallines Melaminpulver nach einem der Ansprüche 1-3, **dadurch gekennzeichnet, daß** die Melam-Konzentration weniger als 0,5 Gew.-% beträgt.

5.  Multikristallines Melaminpulver nach einem der Ansprüche 1-4, **dadurch gekennzeichnet, daß** die Melamin-Reinheit mehr als 99 Gew.-% beträgt.

6.  Multikristallines Melaminpulver nach Anspruch 5, **dadurch gekennzeichnet, daß** die Melamin-Reinheit zwischen 99,5 und 99,8 Gew.-% liegt.

7. Multikristallines Melaminpulver nach einem der Ansprüche 1-6, **dadurch gekennzeichnet, daß** die Schüttdichte (lose) zwischen 450 und 550 kg/m$^3$ liegt.

8. Multikristallines Melaminpulver nach einem der Ansprüche 1-7, **dadurch gekennzeichnet, daß** die Vergilbung des Melaminpulvers (b') weniger als 1 beträgt.

9. Multikristallines Melaminpulver nach Anspruch 8, **dadurch gekennzeichnet, daß** die Vergilbung (b') weniger als 0,8 beträgt.

**Revendications**

1. Poudre de mélamine polycristalline possédant les caractéristiques suivantes :

   - $d_{90}$: 50-150 $\mu$m ; $d_{50}$ < 50 $\mu$m
   - densité apparente (lâche) 430-570 kg/m$^3$
   - couleur APHA inférieure à 17
   - mélamine : > 98,5 % en masse
   - melam : < 1 % en masse

2. Poudre de mélamine polycristalline selon la revendication 1, **caractérisée en ce que** le $d_{90}$ est compris entre 70 et 120 $\mu$m et **en ce que** le $d_{50}$ est inférieur à 40 $\mu$m.

3. Poudre de mélamine polycristalline selon l'une quelconque des revendications 1-2, **caractérisée en ce que** la couleur est inférieure à 15 APHA.

4. Poudre de mélamine polycristalline selon l'une quelconque des revendications 1-3, **caractérisée en ce que** la concentration en melam est inférieure à 0,5 % en masse.

5. Poudre de mélamine polycristalline selon l'une quelconque des revendications 1-4, **caractérisée en ce que** la pureté de la mélamine est supérieure à 99 % en masse.

6. Poudre de mélamine polycristalline selon la revendication 5, **caractérisée en ce que** la pureté de la mélamine est comprise entre 99,5 et 99,8 % en masse.

7. Poudre de mélamine polycristalline selon l'une quelconque des revendications 1-6, **caractérisée en ce que** la densité apparente (lâche) est comprise entre 450 et 550 kg/m$^3$.

8. Poudre de mélamine polycristalline selon l'une quelconque des revendications 1-7, **caractérisée en ce que** le jaunissement de la poudre de mélamine (b') est inférieur à 1.

9. Poudre de mélamine polycristalline selon la revendication 8, **caractérisée en ce que** le jaunissement (b') est inférieur à 0,8.

Fig 1A

1mm15.1kV 5.05E1 3967/23 95-157A

Fig 1B

10µm150kV 1.55E3 3969/23 95-157A

Fig 2A

Fig 2B